# EUROPEAN PATENT APPLICATION

(11) **EP 4 631 526 A1**
(43) Date of publication of application: **15.10.2025**
(21) Application number: 24168910.8
(22) Date of filing: 08.04.2024
(51) Int. Cl.: A61K 47/69

(54) **HETEROBIFUNCTIONAL LINKER**

(71) Applicant: ETH Zurich, 8092 Zurich (CH)
(72) Inventor: BODE, Jeffrey, 8003 Zürich (CH); KEYS, Timothy, 8312 Winterberg (CH); SLACK, Emma, 8105 Watt (CH); RUTSCHMANN, Christoph, 8004 Zürich (CH); SCHILLING, Philipp, 8046 Zürich (CH); PIECH, Lucas, 1030 Wien (AT); TURGAY, Yagmur, 8004 Zürich (CH)

(57) **Abstract**

The present invention relates to a heterobifunctional linker and its use in the preparation of a vaccine.

## Description

The present invention relates to a heterobifunctional linker and its use in the preparation of a vaccine.

Glycoconjugate vaccines are widely recognized as the safest and most effective class of anti-bacterial vaccines. The focus of these vaccines are the distinct polysaccharides that form the primary external characteristics of bacterial cells, specifically the O and K antigens. The O antigens, integral to the lipopolysaccharide (LPS) coat of Gram-negative bacteria, consist of chains of sugar units and are key to the differentiation of bacterial serotypes. K antigens are the capsular polysaccharides encasing some bacteria, providing a protective shell and aiding in the bacteria's ability to resist immune attack. K antigens are considered as heat-and acid-labile structures. In the 1940s, capsular polysaccharides were called "L antigens" for labile antigens, because the serologists realized that they could be removed from cells by heat or mild acid treatment. It was assumed that the antigens were destroyed by these treatments.

These polysaccharides are pivotal in the bacteria's interaction and evasion strategies within the host immune system. Despite their vital role in bacteria and potential as vaccine targets, these polysaccharides alone elicit a weak immune response. To amplify their immunogenicity, glycoconjugate vaccines covalently link a protein, commonly regarded as an immunogenic carrier protein, to the targeted O or K polysaccharide. This combination activates specific immune mechanisms, recruiting T cell help for B cells specific to the polysaccharide. Consequently, this strategy induces a strong production of high-affinity antibodies and secures a robust and long-lived immune memory, preparing the body for a more effective defense against bacterial infections.

Commercially available glycoconjugate vaccines offer robust protection against a quartet of pathogenic encapsulated bacteria: Streptococcus pneumoniae (Sp), Neisseria meningitidis (Nm), Haemophilus influenzae (Hi), and Salmonella typhi. These immunizations are formulated using capsular polysaccharides extracted directly from the bacteria they target and are chemically bound to protein carriers such as tetanus toxoid (TT) or diphtheria toxoid (CRM197). When a particular bacterium has multiple serotypes implicated in diseases, the corresponding vaccines are designed to be multivalent, targeting several polysaccharides. For instance, Pfizer's latest pneumococcal vaccine, Prevnar PCV20, incorporates 20 different polysaccharide conjugates, while meningococcal vaccines from Pfizer, GSK, and Sanofi Pasteur each contain 4 polysaccharide conjugates.

Currently, there are several significant drawbacks associated with state-of-the-art conjugation chemistries that undermine their versatility and practicality. A major issue is that these chemistries lack broad applicability; they must be specifically tailored and adapted for each unique polysaccharide antigen, complicating the development and limiting the use within vaccine design. This specificity in approach is evident in the case of GSK's Nimenrix vaccine, which requires two divergent chemistries to conjugate its four Neisserial K antigens, adding layers of complexity and cost to the vaccine production process. Additionally, the activation step, where reactive groups are introduced to the polysaccharide chains, can be detrimental to the integrity of the antigen. This step risks depolymerizing the chains or damaging key protective epitopes. This particular drawback has materialized in the pneumococcal serotypes 1 and 5 vaccines, where the manipulation of protective epitopes during conjugation has led to notably lower levels of protection from these vaccines. Furthermore, despite the recognition over four decades ago that capsular polysaccharides of many pathogenic Escherichia coli strains (K antigen) are viable protective vaccine antigens, the development of a multivalent vaccine targeting E. coli capsules has not come to fruition. This is largely due to two significant barriers. Firstly, the extensive variety of disease-causing K-antigen serotypes necessitates the creation of a vaccine with a high degree of multivalency, presenting a substantial challenge in vaccine formulation. Secondly, and perhaps more critically, there is a distinct lack of a suitable chemical conjugation strategy capable of uniformly addressing the chemical diversity of K polysaccharides found in E. coli strains. The chemical heterogeneity includes features like uronic acid, ulosonic acid substituents, sugar-phosphate repeat units, as well as teichoic acid-like polymers containing polyol-phosphates. The absence of a one-size-fits-all conjugation method impedes the development of a comprehensive and effective vaccine against the various E. coli K-antigen serotypes.

WO 2013038375 discloses conjugation techniques as alternatives to those previously known, offering potential speed advantages, especially over methods like reductive amination. These methods eliminate the need for 1-ethyl-3-(3-dimethylaminopropyl)carbodiimide (EDAC), thereby reducing the likelihood of undesired side reactions. Furthermore, the document discloses purification processes for intermediate compounds that avoid toxic substances, enhancing overall safety.

In their 2020 publication in the Journal of Biomedical Science, T. W. Chiu and colleagues, in the article "Constructing conjugate vaccine against Salmonella Typhimurium using lipid-A free lipopolysaccharide," mentioned the presence of a Kdo (3-deoxy-D-manno-octulosonic acid) residue situated at the juncture between the core and lipid A of the O antigen which is generally considered to be stable to heat and acidic conditions. (DOI: 10.1186/s12929-020-00681-8). The disclosed process describes the preparation of "polysaccharide-linker-RG' conjugates, followed by reaction of lysines or azides on proteins with the reactive group (RG), respectively. The associated challenges with this approach are the use of multistep synthesis, control of the conjugation level due to the abundance of lysines, and longer reaction times due to the chemistry used.

WO2013169640 discloses a series of tagged saccharides, designed for use in analytical and diagnostic applications. Specifically, the disclosure encompasses modified glycosides with the structure Y-X, where 'Y' denotes a unit that can be a monosaccharide, oligosaccharide, or polysaccharide. These units are connected in either a linear or branched manner through glycosidic linkages. The 'X' in this formula represents a tagging moiety, which consists of an ortho-diaminobenzoic (DAB)-peptide.

The problem of the present invention was to provide a chemical strategy that allows to prepare a vaccine that targets the capsular polysaccharide of Gram-negative pathogens.

The problem is solved by the bifunctional linker according to claim 1. Further preferred embodiments are subject of dependent claims 2 to 15.

It was found that the heterobifunctional linker according to the present invention can be used to attach at one end to the reducing end of a K antigen polysaccharide and at the other end to a protein or a virus-like particle. Thanks to the linker according to the present invention, it is possible to prepare a vaccine that targets the capsular polysaccharide of various Gram-negative pathogens, preferably selected from the group consisting of Actinobacillus pleuropneumoniae, Bibersteinia trehalosi, Bordetella bronchiseptica, Bordetella pertussis, Burkholderia cenocepacia, Burkholderia cepacia, Burkholderia pseudomallei, Campylobacter coli, Campylobacter fetus, Campylobacter lari, Campylobacter jejuni, Citrobacter freundii, Cronobacter sakazakii, Escherichia coli, Haemophilus haemolyticus, Haemophilus influenzae, Haemophilus parainfluenzae, Haemophilus sputorum, Kingella kingae, Klebsiella oxytoca, Klebsiella pneumoniae, Moraxella bovis, Moraxella canis, Moraxella lacunata, Moraxella nonliquefaciens, Moraxella porci, Neisseria meningitidis, Pasteurella multocida, Shigella sonnei, Serratia marcescens, Yersinia enterocolitica, and Yersinia intermedia. A unique feature of the linker is that it allows an end-linked bonding of K antigen polysaccharides. This avoids destruction of protective epitopes along the polysaccharide chain and thus overcomes a major problem of the state-of-the-art conjugation strategies.

K antigens comprise residues that can form alpha-keto-acid on mild acid hydrolysis. Typical residues are ulosonic acids. Preferably said residues are selected from the group consisting of Kdo residues, sialic acids or Kdn residues (2-keto-3-deoxy-D-glycero-D-galacto-nonulosonic acid). Moreover, it has been known for decades that K polymers are unstable under hydrolytic conditions. Surprisingly, it was found that by tuning the conditions, segments of said polysaccharides can be obtained that are shorter than the native polysaccharide but structurally intact. The size of the said segment is, however, greater that 10 kilodaltons, long enough for the intended purpose, i.e. the preparation of a vaccine. Since chemically diverse capsular polysaccharides can be released from their glycolipid anchor by mild acid hydrolysis, for example, at conserved Kdo residues, a polysaccharide with a unique chemical handle (an α-keto acid) at the reducing end is released. This α-keto acid can be used to attach a heterobifunctional linker according to the present invention at the end of the polysaccharide. Reaction of the α-keto acid with the ortho-arylene diamine group A of the bifunctional linker yields a polysaccharide that can be site-specifically conjugated with reactive groups on a carrier protein, that can be part of a virus-like particle. Overall, the bifunctional linker according to the present invention can be synthesized very efficiently and allows to conjugate segments of the capsular polysaccharides to the carrier protein/VLP with very fast kinetics, as in particular the thiol-maleimide reaction is among the most established and practical conjugation methods for attachment to proteins.

The term "virus-like particle" means all of or a part of a virus outer shell protein that mainly constitutes a capsid. The virus-like particle does not raise infection concerns, since the virus-like particle typically does not contain a genome and is therefore not competent to infect cells or reproduce itself. But the virus-like particle can be used as an active ingredient of vaccine, since the virus-like particle causes an immune reaction. In addition, the virus-like particle can be used for a drug delivery system by introducing a synthesized nucleic acid into the virus-like particle or degrading and reconstructing the virus-like particle to enclose a physiologically active substance or the like.

The term "artificial virus" refers to an engineered variant of a virus that has been deliberately designed or modified through biotechnological processes for specific purposes. Unlike naturally occurring viruses, which evolve over time and can cause diseases in humans, animals, or plants, artificial viruses are created in laboratories using genetic engineering techniques.

The bifunctional linker according to the present invention allows in a simple manner to be connected with a multitude of α-keto-acid-containing polysaccharides which is an exceptional advantage in vaccine development.

The heterobifunctional linker L has the general formula (I)

A-X-B₁-Y-B₂-D (I)

or its corresponding salt,
wherein
A is an ortho-arylene diamine group of the formula (II) 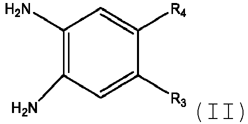
wherein
R₃ is X, or forms together with R₄ a further 6-membered aromatic ring system substituted with X, and if R₃ is X, R₄ is hydrogen,
B₁ and B₂ are independent from each other a spacer group formed by a chain of 1 to 30 covalently linked non-hydrogen atoms selected from the group consisting of C, N, O, and S and composed of any combinations of single, double, triple or aromatic carbon-carbon bonds, carbon-nitrogen bonds, carbon-oxygen bonds, and carbon-sulfur bonds;
D is selected from a cysteine reactive group, a tyrosine reactive group, a methionine reactive group, an alkyne-reactive group and an azide reactive group;
X is a linker group that links A to B₁, whereby X is selected from the group consisting of CONH, NHCO, CONR₅, NR_{5'}CO, COO, OCO, CHR₆, NH, NR₇, O, S, and CH₂, wherein R₅, R_{5'}, R₆ and R₇ are independently selected from C₁ to C₆ linear or branched alkyl, aryl having 6 to 20 carbon atoms in the ring portion and 5 to 6-membered aromatic monocyclic heteroaryl comprising 1, 2, 3 or 4 heteroatoms selected from the group consisting of O, N and S,
Y is a linker group that links B₁, optionally via B₂ to D, whereby Y is selected from the group consisting of a single covalent bond, CONH, NHCO, CONR₈, NR_{8'}CO, COO, OCO, COS, SCO, CHR₉, NH, NR₁₀, O, S, and CH₂, wherein R₈, R_{5'}, R₉ and R₁₀ are independently selected from C₁ to C₆ linear or branched alkyl, aryl having 6 to 20 carbon atoms in the ring portion and 5 to 6-membered aromatic monocyclic heteroaryl comprising 1, 2, 3 or 4 heteroatoms selected from the group consisting of O, N and S.

In the present specification and claims, the following terms apply:
The term "C₁ to C₆ linear or branched alkyl" means straight or branched chain alkyl groups having from 1 to 6 carbon atoms and includes for example, methyl, ethyl, n-propyl, isopropyl, butyl, isobutyl, secondary butyl, tertiary butyl, n-pentyl, isopentyl, neopentyl, n-hexyl, 2-methylpentyl and the like.

As used herein, the term "aryl" refers to an aromatic hydrocarbon group having 6-20 carbon atoms in the ring portion. Typically, aryl is monocyclic, bicyclic or tricyclic aryl having 6-20 carbon atoms. In a preferred embodiment, aryl is phenyl.

As used herein, the term "heteroaryl" refers to a 5- or 6-membered aromatic monocyclic ring radical, which comprises 1, 2, 3 or 4 heteroatoms individually selected from nitrogen, oxygen and sulfur. The heteroaryl radical may be bonded via a carbon atom or heteroatom. Examples of heteroaryl include, but are not limited to, furyl, pyrrolyl, thienyl, pyrazolyl, imidazolyl, thiazolyl, isothiazolyl, oxazolyl, isoxazolyl, triazolyl, tetrazolyl, pyrazinyl, pyridazinyl, pyrimidyl or pyridyl.

The term "cysteine reactive" refers to a characteristic of certain chemical moieties that have the ability to react with cysteine residues in proteins. Examples of cysteine reactive moieties include maleimides, iodoacetamides, disulfides, alkyl halides, vinyl sulfones and acrylamides.

The term "tyrosine reactive" refers to a characteristic of certain chemical moieties that have the ability to react with the aromatic ring or phenol moiety of the tyrosine side chain in proteins. Examples of tyrosine reactive moieties include nitration agents, halogenation agents (such as bromination and iodination reagents), reactive diones (such as p-toluenesulfonylamido-phenyltriazolinedione) and sulfonylation agents. Alternatively, a tyrosine reactive group can also be nucleophile, such as a thiol, which can engage in a reaction with an ortho-quinone that has been generated by the enzymatic oxidation of a tyrosine side chain of a protein (Shimakawa et al, J.Am. Chem. Soc. 2023, 145, 1, 600-609)
The term "methionine reactive" refers to the property of certain chemical moieties that can specifically interact with or modify the sulfur-containing side chain of methionine residues in proteins. Examples of methionine reactive moieties include alkylating agents, oxidizing agents, and reagents that specifically target sulfur atoms for modification.

The term "alkyne reactive" as used herein, refers to a chemical moiety that selectively reacts with an alkyne, such as a terminal alkyne or an activated alkyne, on another molecule to form a covalent chemical bond between the alkyne modified group and the alkyne reactive group. Examples of alkyne-reactive groups include, but are not limited to, azides and nitrones.

The term "azide reactive" as used herein, refers to a chemical moiety that selectively reacts with an azide on another molecule to form a covalent chemical bond between the azido modified group and the azide reactive group. Examples of azide-reactive groups include, but are not limited to, alkynes, including, but not limited to, terminal alkynes and activated alkynes such as dibenzoazacyclooctynes; and phosphines, including, but not limited to, triarylphosphines.

The present invention also includes tautomers in cases where a tautomeric equilibrium is present. The term "tautomeric equilibrium" refers to a condition where two (or more) structural isomers are in a dynamic balance with each other, interconverting through the repositioning of a proton (H⁺) and a shift in the location of double bonds within the molecule.

The presence of the ortho-arylene diamine group of the formula (II) in the bifunctional linker according to the present invention is essential as it allows a specific chemical reaction with the α-keto acid found in K antigen polysaccharides, in particular with K antigen polysaccharides comprising one or more residues selected from the group consisting of Kdo (3-deoxy-D-manno-oct-2-ulosonic acid) residues, sialic acids and Kdn residues (2-keto-3-deoxy-D-glycero-D-galacto-nonulosonic acid). This reaction yields a heteroaromatic compound characterized by its remarkable stability, even within the complex settings of biological systems. Such stability is fundamental for maintaining its structural integrity and functionality within the body, vital aspects for successful vaccine development.

In one embodiment of the present invention A is represented by the general formula (IIA)

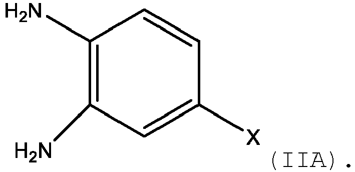

Consequently, in formula (II), with R₃ as X and R₄ as hydrogen, and X defined as earlier stated, the resulting structure is referred to as formula (IIA). This structure facilitates easier synthesis from commercially available building blocks.

In another embodiment of the present invention, A has the general formula (IIB)

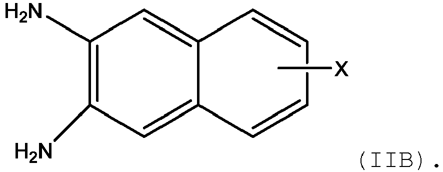

Consequently, in formula (II), R₃ forms together with R₄ a further 6-membered aromatic ring system that is substituted with X, and X has the same definition as above. Linker group X can be located at any arbitrary position on the aromatic ring formed by R₃ and R₄.

Linker group X links A to B₁, whereby X is selected from the group consisting of CONH, NHCO, CONR₅, NR₅'CO, COO, OCO, CHR₆, NH, NR₇, O, S, and CH₂, wherein R₅, R₅', R₆ and R₇ are independently selected from C₁ to C₆ linear or branched alkyl, aryl having 6 to 20 carbon atoms in the ring portion and 5 to 6-membered aromatic monocyclic heteroaryl comprising 1, 2, 3 or 4 heteroatoms selected from the group consisting of O, N and S. Preferably, X is an amide because it can be easily synthesized through the reaction of an amine with either an acid or an ester. This process renders it a versatile and accessible choice for linking A and B₁, with the additional benefit of forming a stable bond.

B₁ and B₂ are independent from each other a spacer group formed by a chain of 1 to 30 covalently linked non-hydrogen atoms selected from the group consisting of C, N, O, and S and composed of any combinations of single, double, triple or aromatic carbon-carbon bonds, carbon-nitrogen bonds, carbon-oxygen bonds, and carbon-sulfur bonds. Thus, B₁ and B₂ are a flexible link between the functional moieties of the bifunctional linker, essentially acting as a bridge. For example, a simple alkylene chain, consisting of carbon atoms connected by single bonds, offers a straightforward, flexible spacer. Another example is a polyethylene glycol (PEG) chain, where repeating units of -CH₂-CH₂-O- introduce both flexibility and solubility in water. These variations allow for the customization of the molecule's characteristics, such as its solubility or the distance between functional groups. Preferably, spacer group B₁ is a C₁ to C₃₀ alkylene or a polyether residue comprising up to 20 carbon atoms and up to 10 oxygen atoms, preferably 1 to 10 polyethylene glycol units. A spacer within this size range offers a favourable combination of flexibility and length. The flexibility allows for movement and proper orientation of attached molecules, which can be critical for interaction with antibodies, while the length ensures the attached molecules are not hampered by steric hindrance, allowing for better access to binding sites. Furthermore, polyethylene glycol units enhance the water solubility of the bifunctional linker according to the present invention. B₂ can be present or absent. Thus, linker group Y links B₁ directly to D or via B₂. Preferably, spacer group B2 is a C₁ to C₁₀ alkylene, preferably a C₂ to C₅ alkylene. The presence of B₂ is often a strategic choice, for enhanced flexibility regarding starting materials. In a preferred embodiment, D is a cysteine reactive group. The cysteine reactive group allows forming a covalent bond with the thiol group of a cysteine residue on the surface of a target protein, a protein of a virus-like particle (VLP) or an artificial virus, facilitating the stable attachment of the bifunctional linker. Preferably, D is selected from the group consisting of 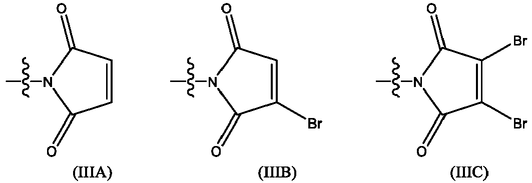 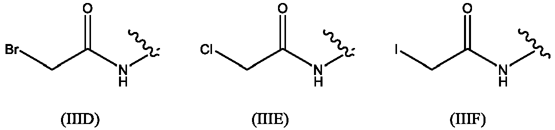 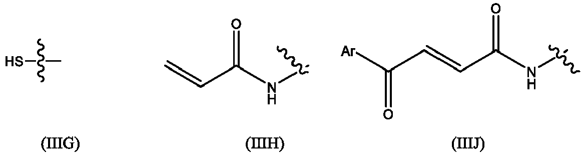 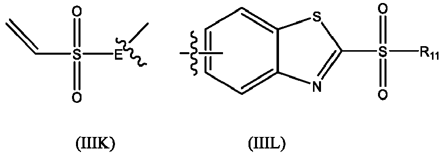 wherein E is a C₁ to C₅ alkylene and R₁₁ is a linear or branched C₁ to C₅ alkyl, which all form stable covalent bonds with the thiol residue of the cysteine. In an especially preferred embodiment, D is selected from the group consisting of residues IIIA, IIIB, IIIC as the thiol-maleimide reaction stands out as one of the fastest in the field of bioconjugation. It also provides enhanced precision in managing the conjugation degree, such as the glycan density, which is beneficial to avoid over-conjugation. In addition, by avoiding NHS esters the stability can be enhanced by reducing the likelihood of polymerization of the heterobifunctional linker over time. Alternatively, D is preferably an alkyne or an azide reactive group each capable of being conjugated to a modified protein through Click chemistry. For this connection to occur, the modified protein should present a complementary reactive group: it must comprise an azide group if D is an alkyne, and conversely, if D holds an azide group, the modified protein should have an alkyne group to ensure a successful reaction. Possible alkyne reactive groups are linear or branched aliphatic azides, aromatic azides and azido-modified amino acids. Possible azide reactive groups are terminal alkynes and activated alkynes, in particular cyclic alkynes such as dibenzoazacyclooctynes and thiocycloalkynes, such as tetra-methylthiacycloheptyne (TMTH), TMTH-SulfoxImine (TMTHSI).

Linker group Y links B₁, optionally via B₂ to D, whereby Y is selected from the group consisting of a single covalent bond, CONH, NHCO, CONR8, NR8'CO, COO, OCO, COS, SCO, CHR9, NH, NR10, O, S, and CH2, wherein R8, R9 and R10 are independently selected from C1 to C6 linear or branched alkyl, aryl having 6 to 20 carbon atoms in the ring portion and 5 to 6-membered aromatic monocyclic heteroaryl comprising 1, 2, 3 or 4 heteroatoms selected from the group consisting of O, N and S.. Preferably, Y is an amide because it can be easily synthesized through the reaction of an amine with either an acid or an ester. This process renders it a versatile and accessible choice for linking B₁ and D, with the additional benefit of forming a stable bond. Often, Y is followed by B₂, preferably 1 to 5 carbon atoms as this facilitates the synthesis of the bifunctional linker. For example in compound (1) 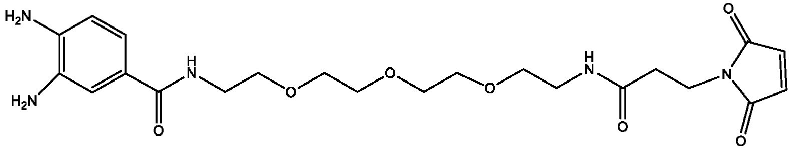 B₁ consists of PEG units, D is a cysteine reactive group of the formula 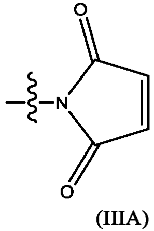 and Y is an amide followed by B2, i.e. an ethylene group. This allows the reaction of an amine functionalized PEG with an activated ester of maleimide.

Preferably, the heterobifunctional linker is selected from the group consisting of compounds 1 to 32 which showed especially good results:

| | |
|---|---|
| 1 | 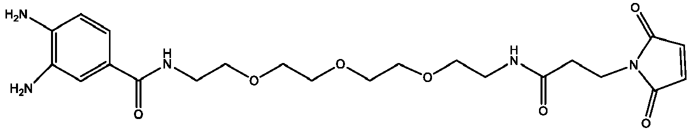 |
| 2 | 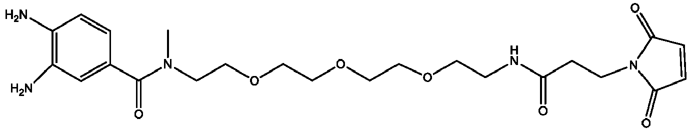 |
| 3 | 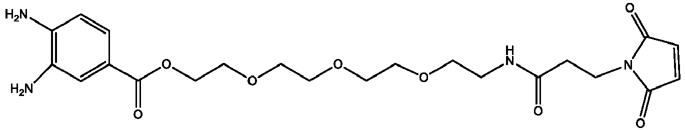 |
| 4 | 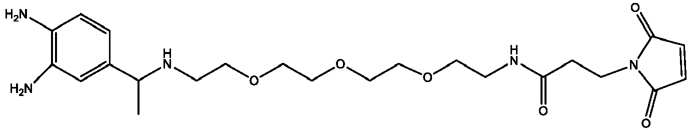 |
| 5 | 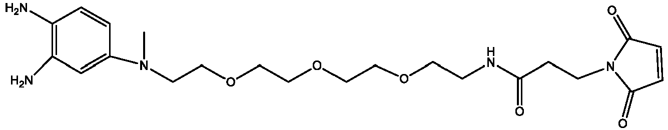 |
| 6 | 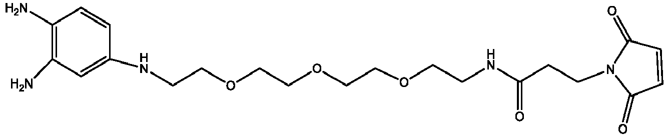 |
| 7 | 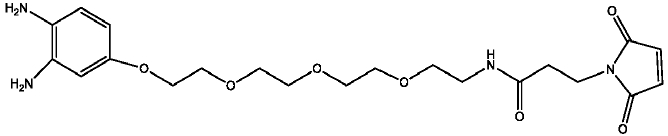 |
| 8 | 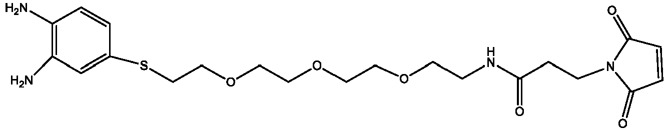 |
| 9 | 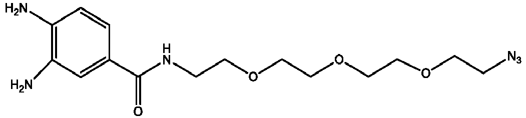 |
| 10 | 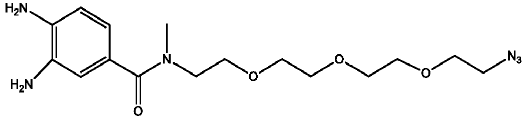 |
| 11 | 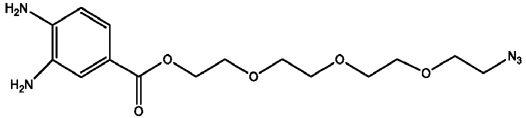 |
| 12 | 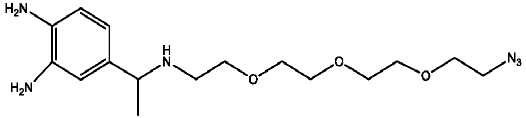 |
| 13 | 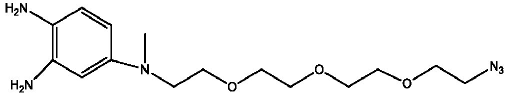 |
| 14 | 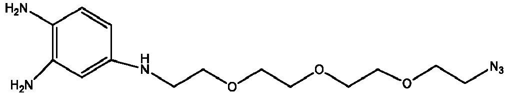 |
| 15 | 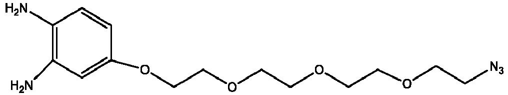 |
| 16 | 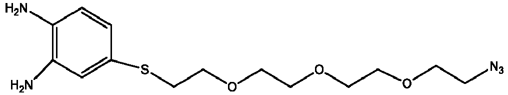 |
| 17 | 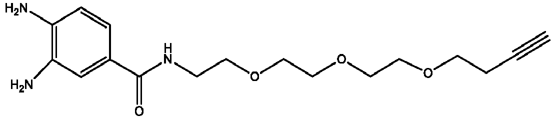 |
| 18 | 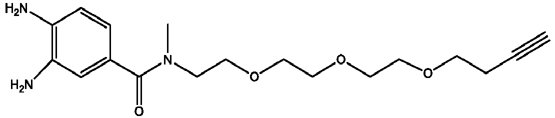 |
| 19 | 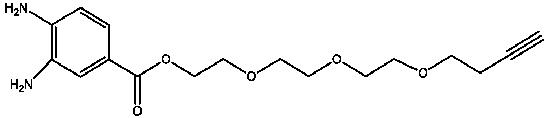 |
| 20 | 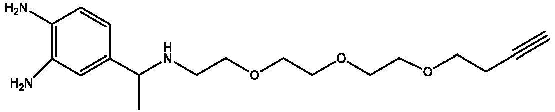 |
| 21 | 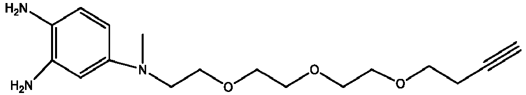 |
| 22 | 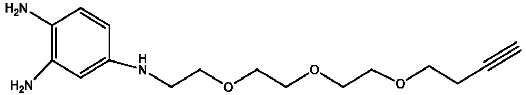 |
| 23 | 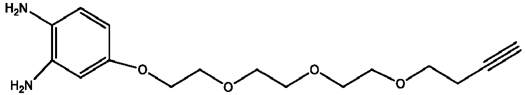 |
| 24 | 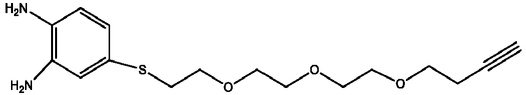 |
| 25 | 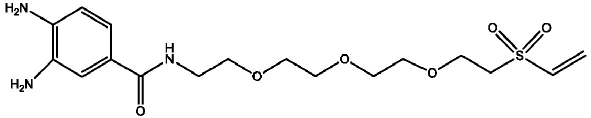 |
| 26 | 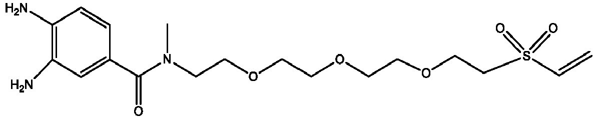 |
| 27 | 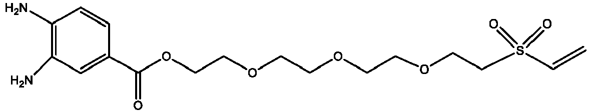 |
| 28 | 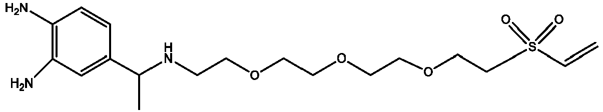 |
| 29 | 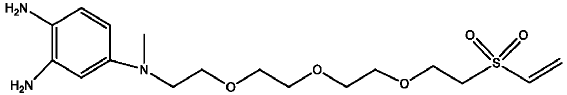 |
| 30 | 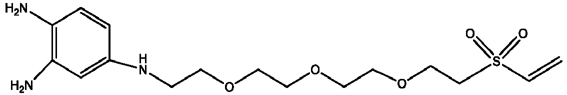 |
| 31 | 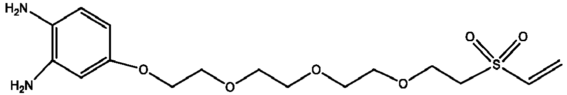 |
| 32 | 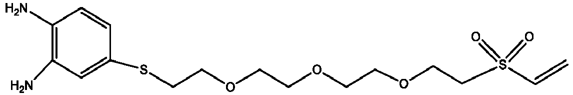 |

A further embodiment of the present invention relates to a conjugate of the formula (IV)

CPS-G-X-B₁-Y-B₂-D (IV)

wherein
CPS is a segment of capsular polysaccharide, wherein an α-keto acid of said segment of the capsular polysaccharide (thus of CPSα) forms with the ortho-arylene diamine group A of the bifunctional linker group G having the general formula (V) 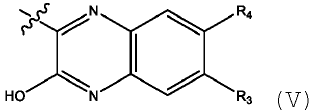
and its tautomeric forms, and
X, B₁, B₂, Y, D, R₃ and R₄ have the same definition as above.

Thus, the reaction of the α-keto acid of CPSα with the ortho-arylene diamine group A of the bifunctional linker 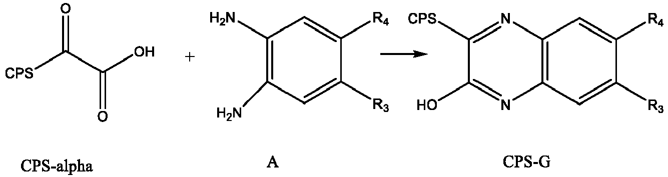 results in a stable heteroaromatic ring system. Further, said heteroaromatic ring system is fluorescent, enabling detection of the functionalized polysaccharide. Through group D, the resulting conjugate (IV) can be site-specifically attached to the reactive group on a carrier protein, that can be part of a virus-like particle or of an artificial virus.

Within the context of the present invention the term "CPSα" stands for a segment of a capsular polysaccharide comprising an alpha-keto-acid. The segment of the capsular polysaccharide comprises at least 2 repeat units, preferably at least 10 kilodaltons, of the original polysaccharide's entirety. The segment of the capsular polysaccharide CPSα is obtained from a capsular polysaccharide that forms an alpha-keto-acid on mild acid hydrolysis. Typical residues that form an alpha-keto-acid on mild acid hydrolysis are ulosonic acids. In particular, it refers to segment of polysaccharides obtained by mild hydrolysis of polysaccharides comprising one or more residues selected from the group consisting of Kdo residues, sialic acids or Kdn residues (2-keto-3-deoxy-D-glycero-D-galacto-nonulosonic acid).

Preferably, CPSα is obtained from capsular proteins selected from the group consisting of K2a and K23 (for details see experimental part).

A further embodiment of the present invention relates to a conjugate of the formula (VI)

A-X-B₁-Y-B₂-H-P (VI)

wherein
P is a protein or a modified protein with reactive group Q,
wherein Q is selected from the thiol group of a cysteine, the side chain of tyrosine, the thioether group of methionine,
the azide reactive group of a modified protein and the alkyne reactive group of a modified protein,
A, X, B₁, B₂ and Y have the same definition as above,
and H is the group formed by reaction between Q and D, with D being as defined as above.

Thus, in case Q is thiol group of a cysteine, D is a cysteine reactive group, they together create a linker group H in the form of a disulfide bond.

H is preferably selected from the group consisting of a disulfide, ester, sulfate, phosphate and 1,4-disubstituted triazoles, preferably disulfide and disubstituted triazoles. Through the ortho-arylene diamine group A, the resulting conjugate (VI) can be site-specifically reacted with the α-keto acid of a CPSα. Preferably, the protein is part of a virus like particle as this configuration significantly enhances the stimulation of both the humoral (B-cell-mediated) and cellular (T-cell-mediated) arms of the adaptive immune system.

A further embodiment of the present invention relates to a conjugate of the formula (VII)

CPS-G-X-B₁-Y-B₂-H-P (VII),

wherein CPS, G, X, B₁, B₂, Y, H and P have the same definition as above. Conjugates of formula (VII) are useful as vaccines against diseases caused by Gram-negative pathogens, preferably Gram-negative pathogens selected from the group consisting of Actinobacillus pleuropneumoniae, Bibersteinia trehalosi, Bordetella bronchiseptica, Bordetella pertussis, Burkholderia cenocepacia, Burkholderia cepacia, Burkholderia pseudomallei, Campylobacter coli, Campylobacter fetus, Campylobacter lari, Campylobacter jejuni, Citrobacter freundii, Cronobacter sakazakii, Escherichia coli, Haemophilus haemolyticus, Haemophilus influenzae, Haemophilus parainfluenzae, Haemophilus sputorum, Kingella kingae, Klebsiella oxytoca, Klebsiella pneumoniae, Moraxella bovis, Moraxella canis, Moraxella lacunata, Moraxella nonliquefaciens, Moraxella porci, Neisseria meningitidis, Pasteurella multocida, Shigella sonnei, Serratia marcescens, Yersinia enterocolitica, and Yersinia intermedia.

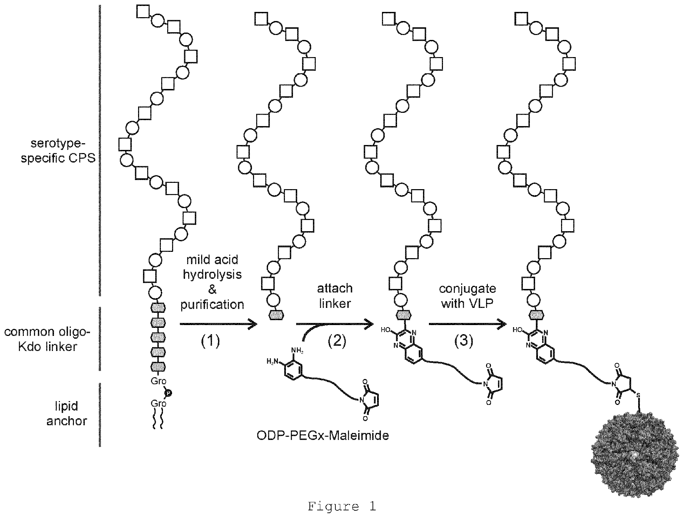

Conjugate (VII) can be prepared by (see Figure 1):
a) Providing a capsular polysaccharide that has been released from a glycolipid anchor,
b) attaching the heterobifunctional linker according to the present invention at the reducing-end of the polysaccharide to obtain a conjugate of the formula (IV), and
c) conjugating the conjugate of the formula (IV) to a carrier protein / a virus like particle.

Alternatively, conjugate (VII) can be prepared by
a) attaching the heterobifunctional linker to a carrier protein / a virus like particle to obtain the conjugate of formula (VI),
b) providing a capsular polysaccharide that has been released from a glycolipid anchor,
c) conjugating the conjugate of formula (VI)to the capsular polysaccharide.

In another aspect of the present invention the conjugate of the formula (VII) is provided as a pharmaceutical composition. A pharmaceutical composition according to the invention includes a conjugate of the formula (VII) as described herein, and may further contain a pharmaceutically acceptable carrier or excipient formulation. The term "pharmaceutically acceptable" is intended to mean a material that is not biologically or otherwise undesirable, which can be administered to an individual along with the conjugate of the formula (VII) without causing significant undesirable biological effects or interacting in a deleterious manner with any of the other components of the pharmaceutical composition in which it is contained. A pharmaceutically acceptable carrier or excipient formulation may include sterile aqueous or nonaqueous solutions, dispersions, suspensions or emulsions, and sterile powders for reconstitution into sterile administrable solutions or dispersions.

A further embodiment of the present invention relates to the use of a bifunctional linker for the preparation of a glycoconjugate vaccine against Gram-negative bacteria. The linker allows the strategic attachment of capsular polysaccharides under mild conditions to proteins, significantly enhancing the immunogenicity of the vaccine. The versatility of this method allows it to be employed in the creation of a broad range of vaccines, catering to various pathogens by enabling the precise targeting of specific immune responses.

A further embodiment of the present invention relates to a method of preparing a vaccine including the following steps:
1) cleavage of capsular polysaccharide segment containing at least one ulosonic acid by hydrolysis from a bacterial strain, resulting in a capsular polysaccharide segment comprising an α-keto acid CPSα,
2) conjugation of said CPSα with the bifunctional linker according to the present invention, and
3) conjugation of the CPS-linker conjugate with a protein or a modified protein with reactive group, whereby the conjugation to the linker can also occur in a different order.

This means that the linker according to the present invention could be first linked to the protein or modified protein, and in a second step this conjugate could be linked to the segment of the capsular polysaccharide comprising an alpha-terminal keto group. These three steps can occur immediately in succession, but it is also possible to store the individual intermediates. Through this modular storage, valuable precursors can be stored.

### Figures

Figure 1 shows the chemical strategy for preparation of glycoconjugate vaccines. Transporter-dependent capsular polysaccharides consist of a diacylglycerol phosphate lipid anchor, a conserved oligosaccharide of Kdo residues (grey hexagons), and the serotype-specific polysaccharide. Mild acid hydrolysis (1) can be used to break glycosidic bonds in the Kdo linker, thereby releasing polysaccharides with a Kdo residue at the reducing end (CPSα). The Kdo at the reducing end provides a unique chemical handle, an α-keto acid group. This group can be used to install (2) the heterobifunctional linker L according to the present invention, at the reducing-end of the polysaccharide, thereby producing a maleimide functionalized polysaccharide. The maleimide group can be used to conjugate (3) the polysaccharide with free-thiol groups on a virus-like particle (VLP), an artificial virus or other carrier protein. The quinoxalinol derivative formed in step (2) is fluorescent, enabling detection of the functionalized polysaccharide.

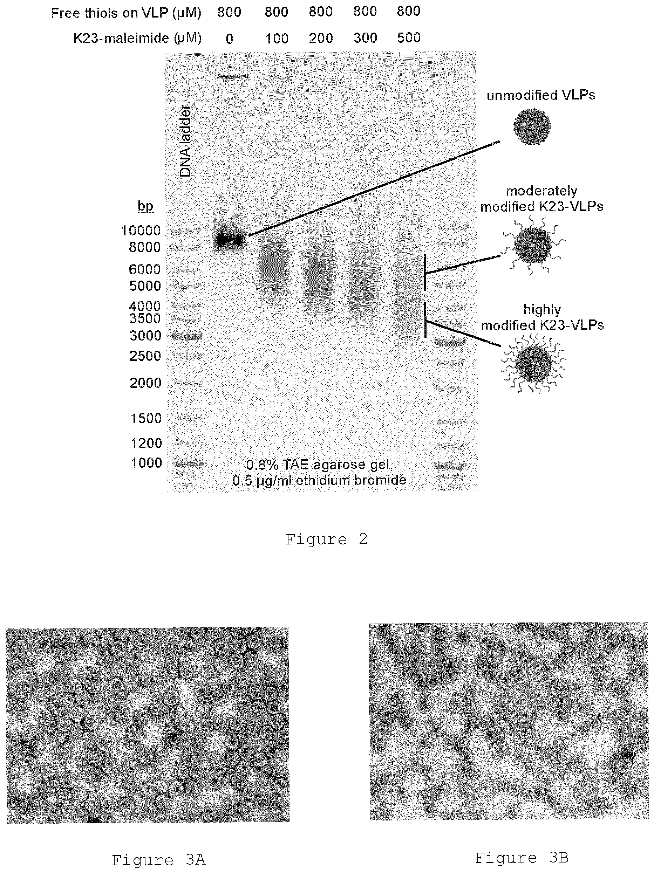

Figure 2 shows the polysaccharide to VLP ratio in the conjugation reaction controls the degree of VLP modification. VLP and maleimide-functionalized K23 polysaccharide were mixed in the indicated ratios. Products from each reaction were analysed by native agarose gel electrophoresis. The high negative charge density of K23 polysaccharide increases the electrophoretic mobility of VLPs.
Figures 3A (Unmodified VLP) and 3B (K23-VLP) show the conjugation with K antigen does not disturb VLP structure. Transmission electron microscopy was used to confirm that VLP structure is not disturbed by conjugation with capsular polysaccharides. Products of the K23-VLP reaction using the highest polysaccharide:VLP ratio were adsorbed on carbon coated copper grids, stained with 2% uranyl acetate, and imaged with the electron microscope. Images showed intact VLPs of comparable size, structure, and abundance to the unmodified VLP control. This data demonstrates that VLP structure is not disturbed by conjugation with capsular polysaccharide.

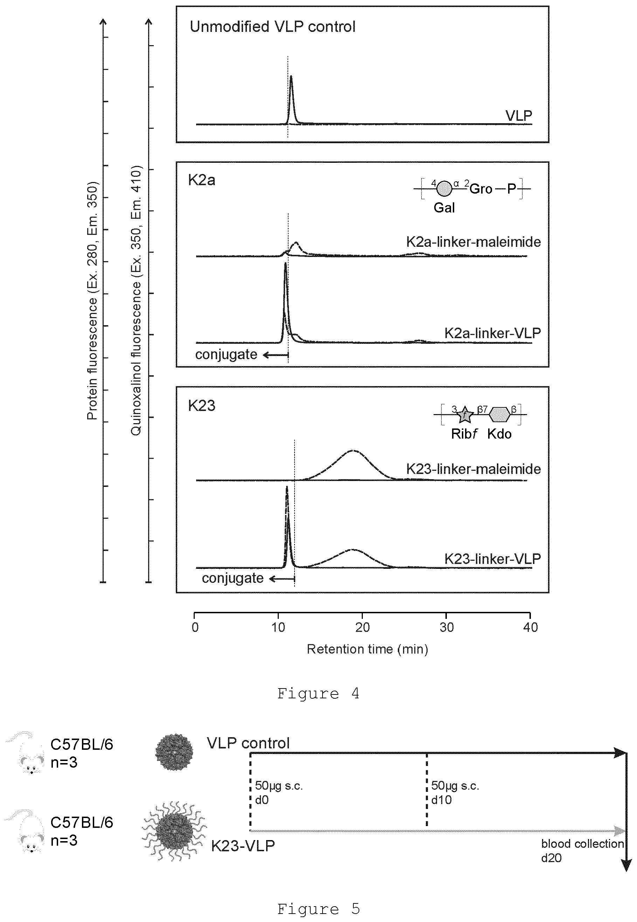

Figure 4 shows the conjugation of E. coli K-antigens with virus-like particles. Capsular polysaccharides were extracted, hydrolysed, and conjugated to the OPD-PEGx-Maleimide linker, i.e. compound of formula 1, re-purified, then conjugated to VLPs. Each panel shows the data for one K-type, including the repeat unit structure (top right), the size-exclusion chromatogram of the maleimide-functionalized polysaccharide (top chromatogram), and the size-exclusion chromatogram of the VLP-conjugated polysaccharide (bottom chromatogram). Solid black lines trace protein fluorescence with a scale of 1×10⁶ arbitrary units per tick mark. Dashed black lines trace fluorescence of the quinoxalinol derivative formed upon reaction of the OPD-PEGx-Maleimide linker with the polysaccharide with a scale of 2.5×10⁴ arbitrary units per tick mark. The top panel displays the chromatograms for unmodified VLP control. Ribf, ribofuranose. Kdo, keto-deoxyoctulosonic acid. Gal, galactose. Gro, glycerol.
Figure 5 shows the vaccination schedule. Mice (n=3 mice per group) were vaccinated subcutaneously on day 0 and day 10 with 50 µg of either K23-VLP or unmodified VLP control. Blood was collected on day 20.

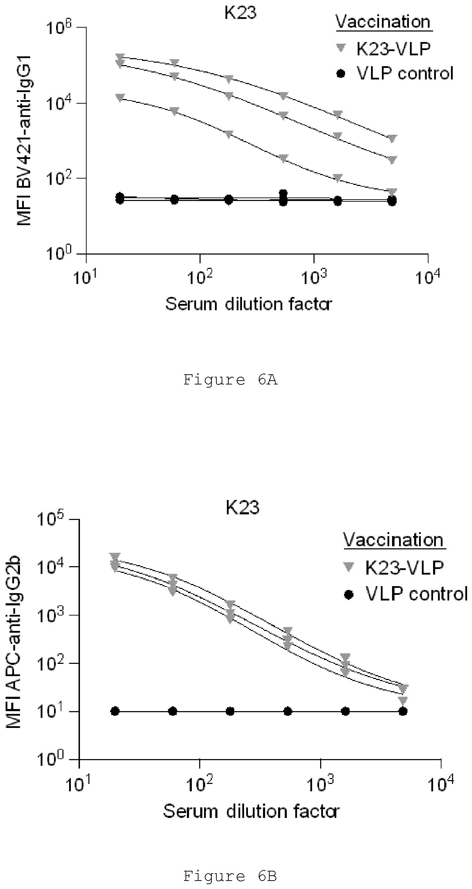

Figures 6A and 6B show that K23-VLP induces IgG1 and IgG2b that bind K23 encapsulated bacteria. Serum from mice vaccinated with K23-VLP or unmodified VLP control were analysed by flow cytometry for IgG1 and IgG2b antibodies that bind to the K23 encapsulated strain E. coli H54.

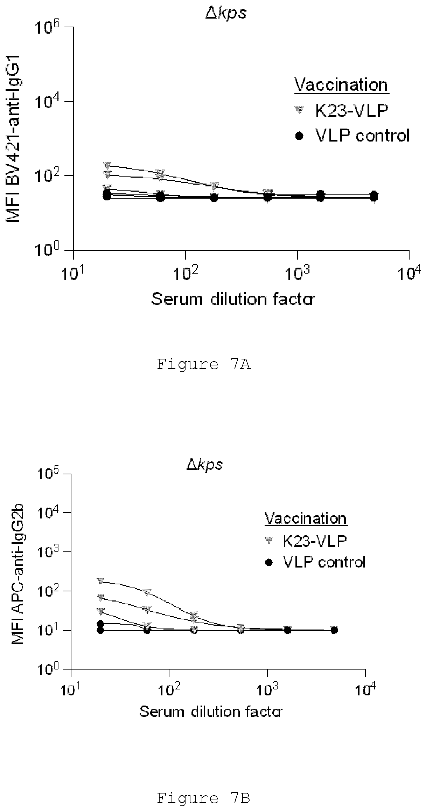

Figures 7A and 7B show that K23-VLP induces small amounts of IgG1 and IgG2b that bind the capsule deletion mutant of E. coli H54. Serum from mice vaccinated with K23-VLP or unmodified VLP control were analysed by flow cytometry for IgG1 and IgG2b antibodies that bind to the capsule deletion mutant Δkps of E. coli H54.

### Examples

### Example 1: OPD-PEG₃-Maleimide linker synthesis

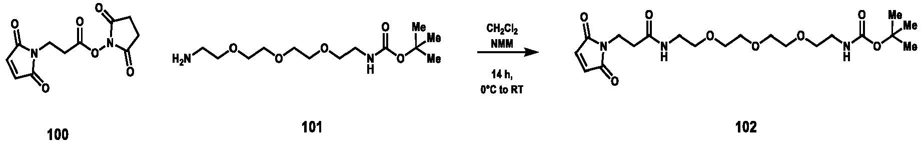

Compound **100** (450 mg, 1.69 mmol, 1 eq) was dissolved in dry CH₂Cl₂ (4.5 mL) and cooled to 0°C in an ice bath with stirring. Compound **101** (544 mg, 1.86 mmol, 1.1 eq) was dissolved in dry CH₂Cl₂ (4 mL) and N-Methylmorpholine was added (0.413 mL, 3.72 mmol, 2.2 eq), the solution was subsequently added to the stirred solution containing compound **100.** The reaction mixture was allowed to warm up to room temperature overnight with stirring. After evaporation of the solvent under reduced pressure, the crude product was dissolved in DMF (1 mL) and H₂O/CH₃CN (7/3, v/v), acidified with acetic acid and purified on a Sfär C18 Duo Reversed Phase column (Biotage) with H₂O/CH₃CN + 0.1% TFA. The product containing fractions were lyophilized, yielding compound **102** as a light-yellow oil (625 mg, 1.41 mmol, 83%). The product was analyzed by NMR and HRMS.

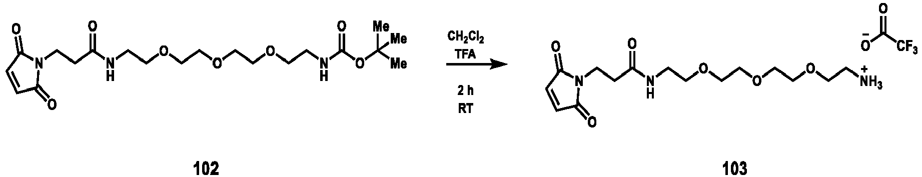

Compound **102** (599 mg, 1.35 mmol) was dissolved in 2 mL CH₂Cl₂ and 2 mL TFA were added. The reaction was stirred for 2 hours at room temperature and evaporated to dryness under reduced pressure and further co-evaporated with toluene (2 × 5 mL). The crude product **103** was directly used in the next step without further purification.

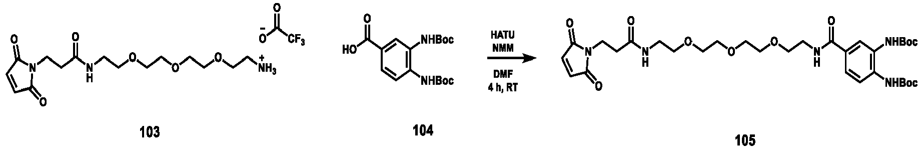

Compound **104** (458 mg, 1.3 mmol, 1 eq) was dissolved in 4 mL DMF. HATU (494 mg, 1.3 mmol, 1 eq) was dissolved in 3 mL DMF and added to compound **104** in DMF. N-Methylmorpholine (0.58 mL, 5.2 mmol, 4 eq) was added and the solution incubated for 5 min at room temperature. Compound **103** (crude product from previous step, 1.35 mmol) was dissolved in 2 mL DMF, N-Methylmorpholine was added (0.867 mL, 7.8 mmol, 6 eq) and subsequently added to the solution containing pre-activated compound **104.** After stirring for 4 hours at room temperature, the reaction was diluted with H₂O/CH₃CN (1/1, v/v), acidified with acetic acid and purified on a Sfär C18 Duo Reversed Phase column (Biotage) with H₂O/CH₃CN + 0.1% TFA. The product containing fractions were lyophilized, yielding compound **105** as a light brown powder (305 mg, 0.45 mmol, 35%). The product was analyzed by NMR and HRMS.

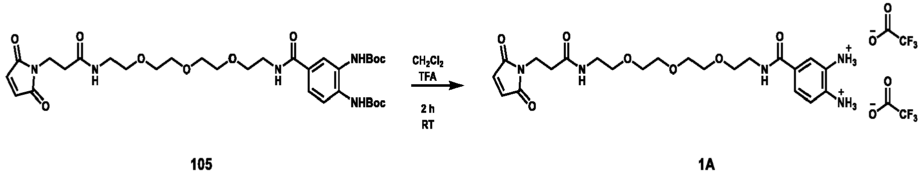

Compound **105** (250 mg, 0.37 mmol) was dissolved in 2 mL CH₂Cl₂ and 2 mL TFA were added. The reaction was stirred for 2 hours at room temperature and evaporated to dryness under reduced pressure. The crude product, which can also be used directly for glycoconjugation, was dissolved in H₂O/CH₃CN (8/2, v/v) and purified on a Sfär C18 Duo Reversed Phase column (Biotage) with H₂O/CH₃CN + 0.1% TFA. The product containing fractions were lyophilized, yielding compound **1A** as a light yellow to brown powder (155 mg, 0.22 mmol, 59% with respect to the bis-TFA salt MW). The product was analyzed by NMR and HRMS.

### Example 2: OPD-PEG₄-DBCO linker synthesis

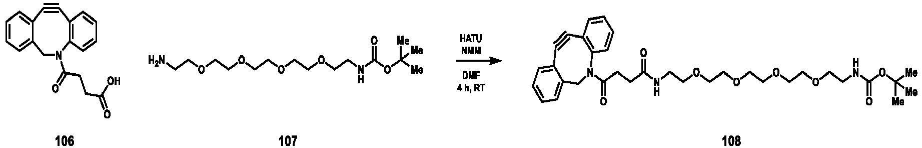

Compound **106** (200 mg, 0.66 mmol, 1 eq) was dissolved in 0.6 mL DMF. HATU (249 mg, 0.66 mmol, 1 eq) was dissolved in 1.5 mL DMF and added to compound **106** in DMF. N-Methylmorpholine (0.183 mL, 1.65 mmol, 2.5 eq) was added and the solution incubated for 5 min at room temperature. Compound **107** (266 mg, 0.79 mmol, 1.2 eq) was dissolved in 1 mL DMF and added to the solution containing pre-activated compound **106.** After stirring for 4 hours at room temperature, the reaction was diluted with H₂O/CH₃CN (1/1, v/v), acidified with acetic acid and purified on a Sfär C18 Duo Reversed Phase column (Biotage) with H₂O/CH₃CN + 0.1% TFA. The product containing fractions were lyophilized, yielding compound **108** (263 mg, 0.42 mmol, 64%). The product was analyzed by NMR and HRMS.

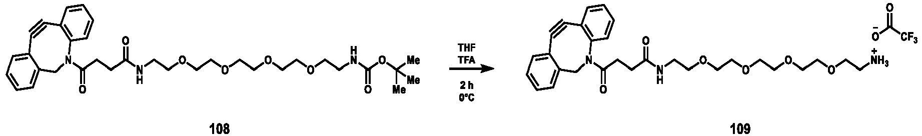

Compound **108** (235 mg, 0.38 mmol) was dissolved in 2 mL THF and 4 mL TFA were added. The reaction was stirred for 2 hours in an ice bath at 0°C and evaporated to dryness under reduced pressure at room temperature and further co-evaporated with toluene (2 × 5 mL). The crude product **109** was directly used in the next step without further purification.

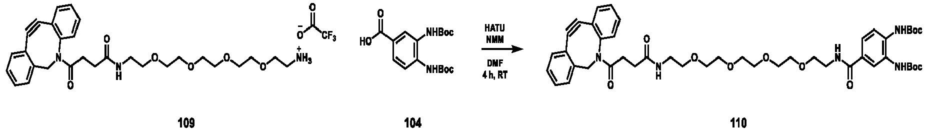

Compound **104** (127 mg, 0.36 mmol, 1 eq) was dissolved in 1 mL DMF. HATU (137 mg, 0.36 mmol, 1 eq) was dissolved in 1 mL DMF and added to compound **104** in DMF. N-Methylmorpholine (0.16 mL, 1.44 mmol, 4 eq) was added and the solution incubated for 5 min at room temperature. Compound **109** (crude product from previous step, 0.38 mmol) was dissolved in 1.5 mL DMF, N-Methylmorpholine was added (0.24 mL, 2.16 mmol, 6 eq) and subsequently added to the solution containing pre-activated compound **104.** After stirring for 4 hours at room temperature, the reaction was diluted with H₂O/CH₃CN (1/1, v/v), acidified with acetic acid and purified on a Sfär C18 Duo Reversed Phase column (Biotage) with H₂O/CH₃CN + 0.1% TFA. The product containing fractions were lyophilized, yielding compound **110** as a light brown powder (128 mg, 0.15 mmol, 41%). The product was analyzed by NMR and HRMS.

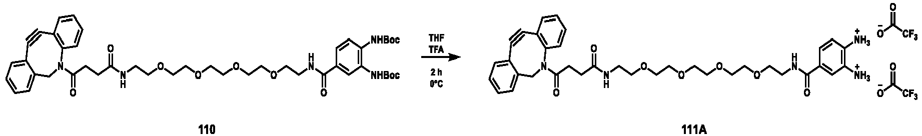

Compound **110** (100 mg, 0.12 mmol) was dissolved in 1 mL THF and 2 mL TFA were added. The reaction was stirred for 2 hours in an ice bath at 0°C and evaporated to dryness under reduced pressure at room temperature. The crude product was dissolved in H₂O/CH₃CN (6/4, v/v) and purified on a Sfär C18 Duo Reversed Phase column (Biotage) with H₂O/CH₃CN + 0.1% TFA. The product containing fractions were lyophilized, yielding compound **111A** as a light-brown powder (52 mg, 0.059 mmol, 49% with respect to the bis-TFA salt MW). The product was analyzed by NMR and HRMS.

### Example 3: Production of K2a-VLP and K23-VLP glycoconjugates

The respective E. coli strains producing K2a and K23 antigen (Table 1) were cultivated at 37°C until stationary phase, while shaking at 180 rpm in Erlenmeyer flasks in Terrific Broth Medium. Cells were washed once with phosphate buffered saline. Lipopolysaccharide and capsular polysaccharide were extracted with hot phenol in 20 mM Tris HCl pH 7.5 and contaminants removed by DNase and RNase digestions. Capsular polysaccharides were separated from lipopolysaccharide by a series of selective precipitations with cetrimonium bromide (CTAB) and ethanol. Capsular polysaccharides were hydrolysed from the lipid anchor in 40 mM trifluoracetic acid using optimized hydrolysis conditions (Table 2), then concentrated and exchanged to water using a 10 kDa molecular weight cutoff ultracentrifugation device. Purified capsular polysaccharides at approximately 100 mg/ml were reacted with 20 mM OPD-PEGx-Maleimide linker in 200 mM sodium acetate buffer pH 3.8, at 37°C for 20 hours. Excess linker was removed with a 10 kDa molecular weight cutoff ultrafiltration device.

Analytical scale conjugation reactions (20 µg VLP) were carried out to demonstrate control of polysaccharide:VLP ratio. Maleimide functionalized K23 polysaccharide was conjugated to virus-like particles (VLPs) with variable molar ratios (1:8, 1:4, 3:8, or 5:8) of polysaccharide-maleimide to free thiol residues. Reactions proceeded at 25°C in phosphate buffered saline for three hours. Samples from each reaction corresponding to 10 µg of VLP were analysed by native agarose gel electrophoresis, using a 0.8% agarose gel in TAE buffer supplemented with 0.5 ug/ml ethidium bromide to stain nucleic acids encapsulated in the virus-like particle (Figure 2). Unmodified VLP has an electrophoretic mobility comparable to DNA of ~9000 base pairs (bp). Conjugation with the polyanionic K23 polysaccharide increases charge density on the VLP, thereby increasing electrophoretic mobility similar to that of DNA of ~3500 bp. The results demonstrate that VLPs can be modified with varying amounts of polysaccharide by controlling the polysaccharide:VLP ratio in the conjugation reaction.

We used transmission electron microscopy to confirm that VLP structure is not disturbed by conjugation with capsular polysaccharides. Products of the K23-VLP reaction using the highest polysaccharide:VLP ratio were adsorbed on carbon coated copper grids, stained with 2% uranyl acetate, and imaged with the electron microscope. See Figures 3A (unmodified VLP) and 3B (K23-VLP) . Images showed intact VLPs of comparable size, structure, and abundance to the unmodified VLP control. This data demonstrates that VLP structure is not disturbed by conjugation with capsular polysaccharide.

Bench-scale conjugation reactions (3 mg VLP) were carried out to produce vaccine for mouse immunization studies. K2a and K23 polysaccharides were conjugated to virus-like particles with a 1:1 ratio of maleimide functionalized polysaccharide to free thiol residues (-0.75 mM equimolar) at 25°C for three hours in phosphate buffered saline. Samples of each functionalized polysaccharide and the conjugation products were analysed by size exclusion chromatography (Figure 4). Functionalized polysaccharides were detected by fluorescence of the quinoxalinol derivative formed upon reaction of OPD with Kdo. VLPs are detected by protein fluorescence. Glycoconjugate product is indicated by co-localization of the polysaccharide and VLP signals at high molecular weight (low retention time) following conjugation.

**Table 1: Strains**

| **K type** | **Strain** | **Reference** | **Source** |
|---|---|---|---|
| K2a | E. coli Bi 7458/41 | J.C. Larsen, etal., " Crossed immunoelectrophoresis and chemical structural analysis used for characterization of two varieties ofEscherichia coli K2 polysaccharide antigen." Medical Microbiology and Immunology, vol. 168, pp. 191-200, 1980, doi: 10.1007/BF02122853 | CCUG 27 |
| K23 | E. coli H54 (WHO test strain for K23) | W. Vann, etal., "Serological, chemical, and structural analyses of the Escherichia coli cross-reactive capsular polysaccharides K13, K20, and K23." Infection and Immunity, vol. 39, no. 2, pp. 623-629, 1983, doi: 10.1128/iai.39.2.623-629.1983 | NCTC 10430 |

**Table 2: Capsular polysaccharide hydrolysis conditions**

| **Polysaccharide** | | **Temperature** | **Time** |
|---|---|---|---|
| K2a | | 60°C | 4 hours |
| K23 | | 40°C | 2 hours |

## Claims

1. Heterobifunctional linker L of the formula (I)
A-X-B₁-Y-B₂-D (I)
or its corresponding salt,
wherein
A is an ortho-arylene diamine group of the formula (II) 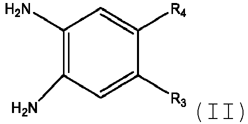
wherein
R₃ is X, or forms together with R₄ a further 6-membered aromatic ring system substituted with X, and if R₃ is X, R₄ is hydrogen,
B₁ and B₂ are independent from each other a spacer group formed by a chain of 1 to 30 covalently linked non-hydrogen atoms selected from the group consisting of C, N, O, and S and composed of any combinations of single, double, triple or aromatic carbon-carbon bonds, carbon-nitrogen bonds, carbon-oxygen bonds, and carbon-sulfur bonds, and B₂ can be present or absent;
D is selected from a cysteine reactive group, a tyrosine reactive group, a methionine reactive group, an alkyne-reactive group and an azide reactive group;
X is a linker group that links A to B₁, whereby X is selected from the group consisting of CONH, NHCO, CONR₅, NR_{5'}CO, COO, OCO, CHR₆, NH, NR₇, O, S, and CH₂, wherein R₅, R_{5'}, R₆ and R₇ are independently selected from C₁ to C₆ linear or branched alkyl, aryl having 6 to 20 carbon atoms in the ring portion and 5 to 6-membered aromatic monocyclic heteroaryl comprising 1, 2, 3 or 4 heteroatoms selected from the group consisting of O, N and S;
Y is a linker group that links B₁, optionally via B₂ to D, whereby Y is selected from the group consisting of a single covalent bond, CONH, NHCO, CONR₈, NR_{8'}CO, COO, OCO, COS, SCO, CHR₉, NH, NR₁₀, O, S, and CH₂, wherein R₈, R₉ and R₁₀ are independently selected from C₁ to C₆ linear or branched alkyl, aryl having 6 to 20 carbon atoms in the ring portion and 5 to 6-membered aromatic monocyclic heteroaryl comprising 1, 2, 3 or 4 heteroatoms selected from the group consisting of O, N and S.

2. Heterobifunctional linker according to claim 1, wherein A is selected from the group consisting of

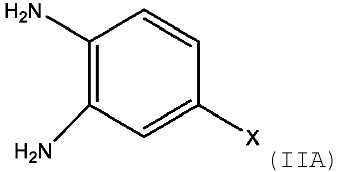

3. Heterobifunctional linker according to any of the preceding claims, wherein the spacer group B₁ is a C₁ to C₃₀ alkylene or a polyether residue comprising up to 20 carbon atoms and up to 10 oxygen atoms, preferably 1 to 10 polyethylene glycol units.

4. Heterobifuctional linker according to any of the preceding claims, wherein the spacer group B₂ is a C₁ to C₁₀ alkylene, preferably a C₂ to C₅ alkylene.

5. Heterobifunctional linker according to any of the preceding claims, wherein D is a cysteine reactive group, preferably selected from the group consisting of 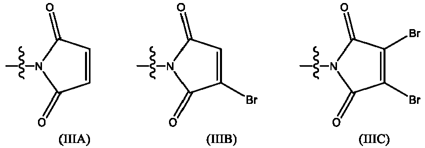 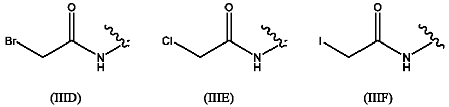 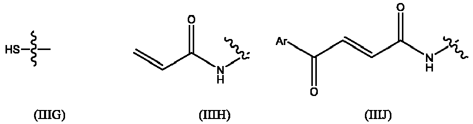 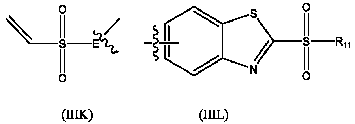 wherein E is a C₁ to C₅ alkylene and R₁₁ is a linear or branched C₁ to C₅ alkyl.

6. Heterobifunctional linker according to any of claims 1 to 4, wherein D is an alkyne or an azide reactive group.

7. Heterobifunctional linker according to any of the preceding claims selected from the group consisting of
| | |
|---|---|
| 1 | 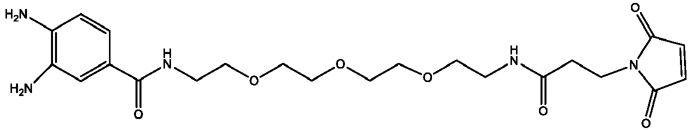 |
| 2 | 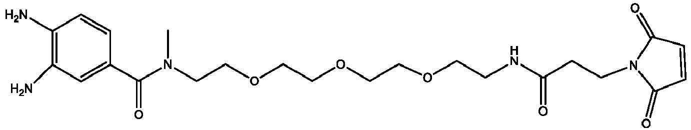 |
| 3 | 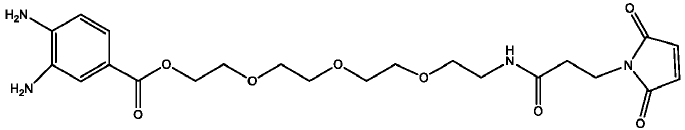 |
| 4 | 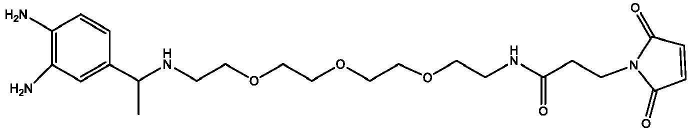 |
| 5 | 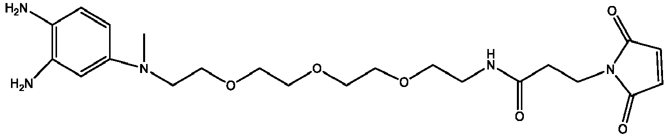 |
| 6 | 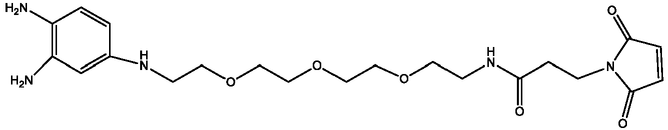 |
| 7 | 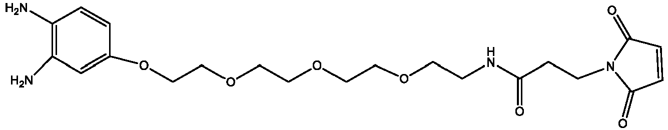 |
| 8 | 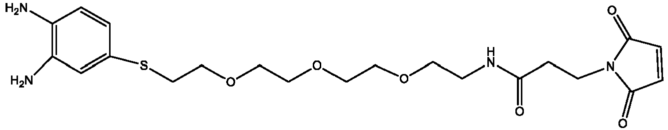 |
| 9 | 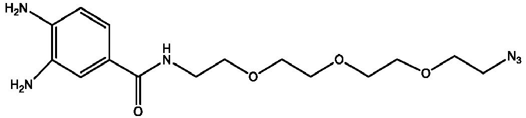 |
| 10 | 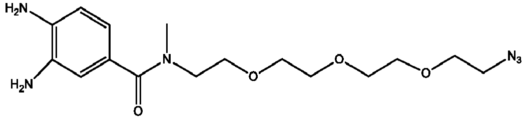 |
| 11 | 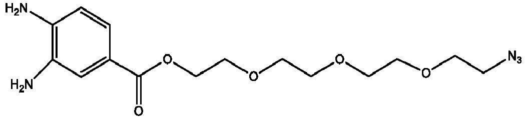 |
| 12 | 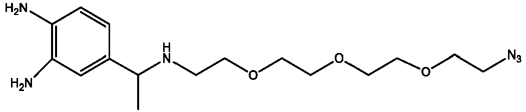 |
| 13 | 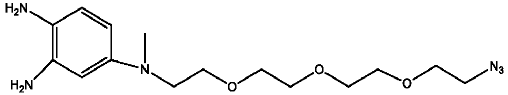 |
| 14 | 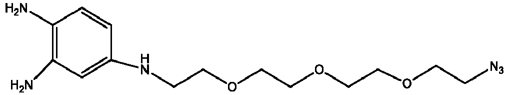 |
| 15 | 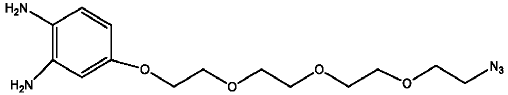 |
| 16 | 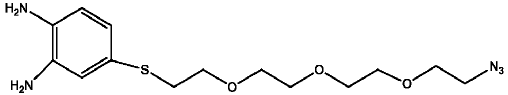 |
| 17 | 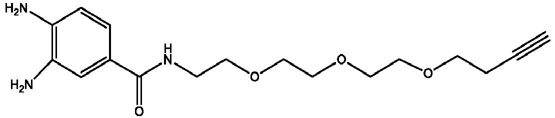 |
| 18 | 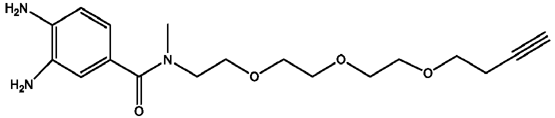 |
| 19 | 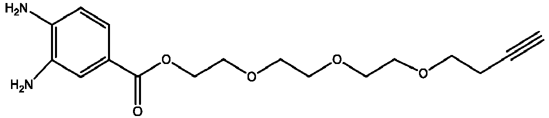 |
| 20 | 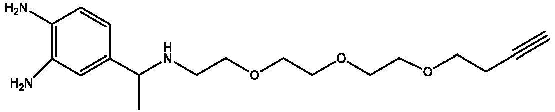 |
| 21 | 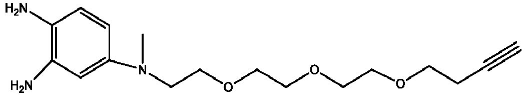 |
| 22 | 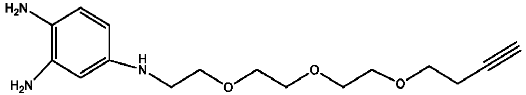 |
| 23 | 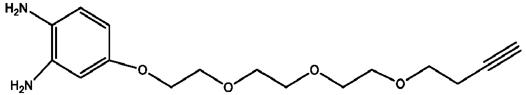 |
| 24 | 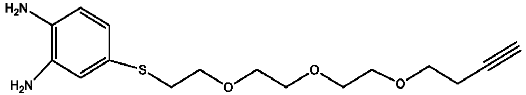 |
| 25 | 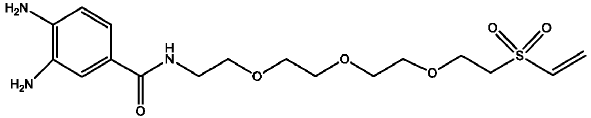 |
| 26 | 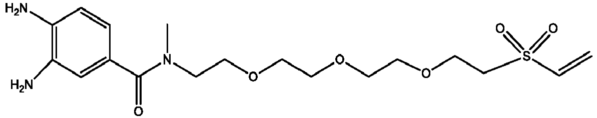 |
| 27 | 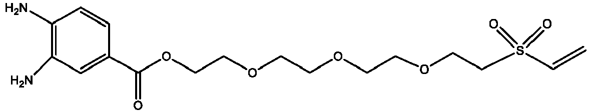 |
| 28 | 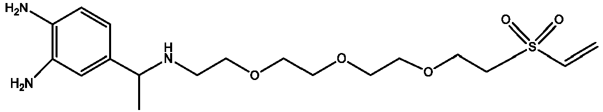 |
| 29 | 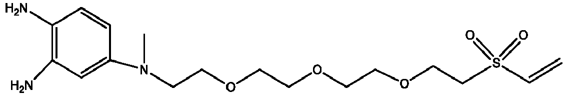 |
| 30 | 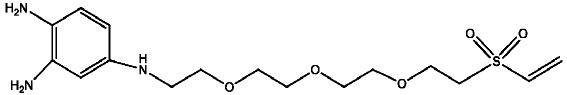 |
| 31 | 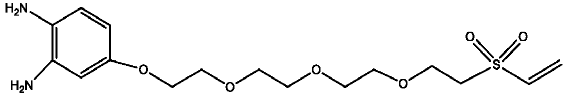 |
| 32 | 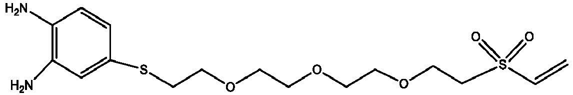 |

8. Conjugate of the formula IV
CPS-G-X-B₁-Y-B₂-D (IV)
wherein CPS is a segment of a capsular polysaccharide, wherein an α-keto acid of said segment of the capsular polysaccharide forms with the ortho-arylene diamine group A of the bifunctional linker group G having the general formula (V) 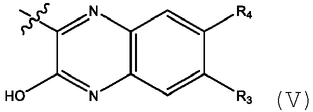 and its tautomeric forms,
X, B₁, B₂, Y, D, R₃ and R₄ have the same definition as in the preceding claims.

9. Conjugate of the formula (VI)
A-X-B₁-Y-B₂-H-P (VI)
wherein
P is a protein or a modified protein with reactive group Q, wherein Q is selected from the thiol group of a cysteine, the hydroxyl group of a tyrosine, the thioether group of methionine, the azide reactive group of a modified protein and the alkyne reactive group of a modified protein,
A, X, B1, B₂ and Y have the same definition as in the preceding claims,
and H is the group formed by the reaction between Q and D, with D being as defined in any of the preceding claims.

10. Conjugate according to claim 9, wherein the protein is part of a virus-like particle or of an artificial virus.

11. Conjugate of the formula (VII)
CPS-G-X-B₁-Y-B₂-H-P (VII),
wherein CPS, G, X, B₁, B₂, Y, H and P have the same definition as in any of the preceding claims.

12. Conjugate of the formula (VII) according to claim 11 for use as a vaccine against Gram-negative bacteria.

13. Conjugate of the formula (VII) according to claim 12 for use as a vaccine against Gram-negative pathogens selected from the group consisting of , preferably selected from the group consisting of Actinobacillus pleuropneumoniae, Bibersteinia trehalosi, Bordetella bronchiseptica, Bordetella pertussis, Burkholderia cenocepacia, Burkholderia cepacia, Burkholderia pseudomallei, Campylobacter coli, Campylobacter fetus, Campylobacter lari, Campylobacter jejuni, Citrobacter freundii, Cronobacter sakazakii, Escherichia coli, Haemophilus haemolyticus, Haemophilus influenzae, Haemophilus parainfluenzae, Haemophilus sputorum, Kingella kingae, Klebsiella oxytoca, Klebsiella pneumoniae, Moraxella bovis, Moraxella canis, Moraxella lacunata, Moraxella nonliquefaciens, Moraxella porci, Neisseria meningitidis, Pasteurella multocida, Shigella sonnei, Serratia marcescens, Yersinia enterocolitica, and Yersinia intermedia.

14. Use of a bifunctional linker according to any of claims 1 to 7 for the preparation of a glycoconjugate vaccine against Gram-negative bacteria.

15. Method of preparing a vaccine including the following steps:
1) cleavage of capsular polysaccharide segment containing at least one ulsonic acid by hydrolysis from a bacterial strain, resulting in a capsular polysaccharide segment comprising an α-keto acid CPSα,
2) conjugation of said CPSα with the bifunctional linker according to claims 1 to 7,
3) conjugation of the CPS-linker conjugate with a protein or a modified protein with reactive group, whereby the conjugation to the linker can also occur in a different order.
